# EUROPÄISCHE PATENTSCHRIFT

(11) **EP 4 436 642 B1**
(45) Veröffentlichungstag und Bekanntmachung des Hinweises auf die Patenterteilung: **08.04.2026**
(21) Anmeldenummer: 22813083.7
(22) Anmeldetag: 10.11.2022
(51) Int. Cl.: A61M 16/00

(54) **SYSTEM ZUR UNTERSTÜTZUNG DES PULMONALEN GASAUSTAUSCHS BEI PATIENTEN**
SYSTEM FOR SUPPORTING THE PULMONARY GAS EXCHANGE IN PATIENTS
SYSTÈME DE PRISE EN CHARGE DE L'ÉCHANGE DE GAZ PULMONAIRE CHEZ DES PATIENTS

(30) Priorität: 14.11.2021 DE 102021005641
(43) Veröffentlichungstag der Anmeldung: 02.10.2024
(73) Patentinhaber: Gründler GmbH, 72250 Freudenstadt (DE)
(72) Erfinder: GRÜNDLER, Markus, 72250 Freudenstadt (DE); GRÜNDLER, Christoph, 72250 Freudenstadt (DE); RESLO, Bernd, 86899 Landsberg am Lech (DE); JOOST, Thilo, 61169 Friedberg (Hessen) (DE); KÖBRICH, Rainer, 68809 Neulußheim (DE)
(86) Internationale Anmeldenummer: PCT/DE2022/000111
(87) Internationale Veröffentlichungsnummer: WO 2023/083400

(56) Entgegenhaltungen:
- EP-A1- 1 329 238
- WO-A1-2005/049124
- DE-A1- 102017 006 655
- DE-A1- 102018 007 517

## Beschreibung

Die Erfindung betrifft ein System zur Unterstützung des pulmonalen Gasaustauschs bei Patienten mit den Merkmalen des Oberbegriffs des Anspruchs 1.

Aus der Druckschrift EP 1329 238 A1 ist eine Vorrichtung für Beatmungssysteme bekannt, wobei die Vorrichtung dazu bestimmt ist, den Totraum im Beatmungssystem zu verringern, und ein erstes Rohr umfasst, das mit dem Totraum im Beatmungssystem verbunden werden kann, um einen Strömungsweg für den Transport von Gas aus dem Totraum im Beatmungssystem bereitzustellen, eine mit dem ersten Schlauch verbundene Saugvorrichtung zum Erzeugen eines einstellbaren Unterdrucks im ersten Schlauch, einen mit dem Totraum im Beatmungssystem verbindbaren zweiten Schlauch um einen Strömungsweg für den Transport von Gas zum Totraum im Beatmungssystem bereitzustellen, eine mit dem zweiten Schlauch verbundene Pumpeinrichtung zum Erzeugen eines einstellbaren Überdrucks im zweiten Schlauch und eine Steuereinheit zum Regeln der Absaugvorrichtung und der Pumpeinrichtung. Die Vorrichtung ist so ausgelegt, dass die Saugvorrichtung und die Pumpvorrichtung aus einer ersten Kammer bzw. einer zweiten Kammer in einem Gehäuse bestehen, die durch eine gasdichte, bewegliche Trennwand voneinander getrennt sind, wobei die Steuereinheit dazu ausgelegt ist, die bewegliche Trennwand bei der Regelung der Saugvorrichtung und der Pumpvorrichtung zu steuern, um einen einfacheren und zuverlässigeren Betrieb zu erreichen.

Aus der Druckschrift DE 10 2017 006 655 A1 ist ein System bekannt zur Unterstützung des pulmonalen Gasaustauschs bei beatmeten Patienten. Die Druckschrift schlägt vor, ein Beatmungssystem, wie es seit vielen Jahren im Einsatz ist, durch ein Totraum-Minimierungssystem zu ergänzen. Dieses Totraum-Minimierungssystem weist zwei Pumpeinheiten auf, wobei eine der beiden Pumpeinheiten einen flexiblen Schlauch aufweist, der über eine Schnittstelle in die Trachealkanüle oder den Endotrachealtubus des Beatmungssystems tief in die Luftröhre des Patienten eingeführt wird. Mittels der zweiten Pumpeinheit wird end-exspiratorisch Atemgas abgesaugt und dieses dann wieder beginnend-exspiratorisch rückgeführt. Eine erste der beiden Pumpeinheiten des Totraum-Minimierungssystems ist patientenseitig vor dem Y-Stück des Beatmungssystems über einen weiteren flexiblen Schlauch angeschlossen und saugt beziehungsweise pumpt zeitlich etwas versetzt, aber im Übrigen gegenläufig zur zweiten Pumpeinheit. Das Totraum-Minimierungssystem bewirkt, dass CO2-reiches verbrauchtes Atemgas besser aus dem Patienten zum Beatmungsgerät und sauerstoffreiche bzw. CO2-arme Frischluft besser vom Beatmungsgerät zum Patienten gelangt.

Ein derartiges System ist bei kleineren Patienten, beispielsweise Kindern, jedoch nur mit Einschränkungen einsetzbar, weil der in die Luftröhre des Patienten reichende Beatmungsschlauch des Beatmungssystems den flexiblen Schlauch der zweiten Pumpeinheit aufnimmt. Die Schläuche können zwar für Kinder kleiner ausgeführt werden, doch ist zur Führung ausreichender Volumenströme ein Mindest-Querschnitt des flexiblen Schlauchs und des Schlauchs des Beatmungssystems erforderlich, der für kleinere Kinder immer noch zu groß sein kann. Insbesondere durch die reine Querschnittsverringerung aber auch durch seine Form und unbestimmte Lage sorgt der flexible Schlauch in dem Beatmungsschlauch für einen erheblichen Strömungswiderstand, so dass es Fälle geben kann, wo zwar eine Totraum-Minimierung wünschenswert wäre, ein zusätzlicher Widerstand im Beatmungsschlauch allerdings medizinisch nicht akzeptabel ist. Dies sind beispielsweise Setups, bei denen der außerhalb des Patienten befindliche Totraumanteil des Beatmungssystems einen relevanten Anteil hat, beispielsweise beim Einsatz von sogenannten Wärmetauscher/Filtern (HME(F) Heat and Moisture Exchanger Filter), und ein solcher beispielsweise zum Infektionsschutz für das behandelnde Personal erforderlich ist.

Der Erfindung liegt daher die Aufgabe zugrunde, ein insbesondere für Kinder besser geeignetes System zur Unterstützung des pulmonalen Gasaustauschs zu schaffen, das den Einsatz eines Filters ohne den Nachteil des dadurch erhöhten Totraums erlaubt.

Diese Aufgabe wird erfindungsgemäß durch das System mit den Merkmalen des Anspruchs 1 gelöst. Das erfindungsgemäße System dient der Unterstützung des pulmonalen Gasaustauschs bei Patienten, wobei die Patienten insbesondere Kinder sein können, jedoch auch Erwachsene. Auch ist die Behandlung von Tieren möglich.

Das System weist ein Beatmungssystem auf, das ein Beatmungsgerät, ein Y-Stück, einen Filter und ein Patienteninterface beinhaltet. Mit "Y-Stück" sind neben einer üblichen Y-förmigen Verzweigung auch andere Elemente mit gleicher Funktion, wie beispielsweise ein Koaxialschlauch mit integrierter Y-Verbindung, gemeint. Darüber hinaus kann das Beatmungssystem weitere Komponenten, wie fluidische Verbindungselemente, aufweisen. Der Filter ist insbesondere ein Filter mit HME-Funktion. Das Patienteninterface ist die Schnittstelle des Beatmungssystems zum Patienten, also im Falle einer invasiven Beatmung insbesondere eine Trachealkanüle oder ein Endotrachealtubus oder im Falle einer nicht-invasiven Beatmung insbesondere eine Gesichtsmaske oder ein Beatmungshelm.

Weiterhin weist das System ein mit dem Beatmungssystem fluidisch gekoppeltes Totraum-Minimierungssystem auf. Insbesondere handelt es sich um ein Totraum-Minimierungssystem wie es für den beatmeten Patienten in der Druckschrift DE 10 2017 006 655 A1 umfassend beschrieben ist. Soweit Abweichungen bestehen, wird nachfolgend hierauf hingewiesen.

Das Totraum-Minimierungssystem weist zwei separat steuerbaren Pumpeinheiten mit je einer Reservoireinheit auf. Die Pumpeinheiten und Reservoireinheiten können insbesondere gemeinsam durch eine Kolbenpumpe gebildet sein. Die fluidische Verbindung zum Beatmungssystem erfolgt insbesondere über flexible Schläuche.

Das Totraum-Minimierungssystem weist außerdem eine Steuerung derart auf, dass eine exspiratorische, insbesondere end-exspiratorische, Absaugung von Atemgas und eine inspiratorische, insbesondere end-inspiratorische, oder beginnend-exspiratorische Rückführung des abgesaugten Atemgases durch eine zweite der beiden Pumpeinheiten einstellbar ist und die erste der beiden Pumpeinheiten im Wesentlichen gegenläufig zur zweiten Pumpeinheit arbeitet. Da beide Pumpeinheiten fluidisch mit dem Beatmungssystem gekoppelt sind, hat dies, wie in der Druckschrift DE 10 2017 006 655 A1 beschrieben, den Vorteil, dass es im Bereich des Beatmungssystems zu keinen störenden Veränderungen der Druck- und Flussverläufe durch das System kommt. Für das Beatmungssystem bleibt das erfindungsgemäße System somit "unsichtbar" und das Beatmungssystem kann ohne Anpassungen betrieben werden. "Im Wesentlichen gegenläufig" schließt dabei ein, dass es Abweichungen von einem exakten Gegenlauf gibt. Diese sind auch zumindest in bestimmten Betriebsmodi gewünscht, was nur durch die separat steuerbaren Pumpeinheiten möglich ist.

Die erste Pumpeinheit ist im Bereich zwischen dem Beatmungsgerät und dem Filter, insbesondere zwischen dem Y-Stück und dem Filter, fluidisch an das Beatmungssystem angeschlossen. "Fluidisch angeschlossen" meint, dass ein fluidischer Strom, insbesondere ein Gasstrom, an dieser Stelle von dem Beatmungssystem in das Totraum-Minimierungssystem und umgekehrt fließen kann.

Das erfindungsgemäße System ist dadurch gekennzeichnet, dass die zweite Pumpeinheit im Bereich zwischen dem Filter und dem Patienteninterface fluidisch angeschlossen ist und somit kein Schlauch des Totraum-Minimierungssystems durch die Luftröhre geführt werden muss. Hierdurch braucht bei einer Gesichts- oder Larynxmaske gar kein Schlauch in die Luftröhre geführt werden, und bei einer Trachealkanüle, einem Endotrachealtubus oder einem Larynxtubus kann ein kleinerer Querschnitt der Schläuche gewählt werden, als dies bei dem in der Druckschrift DE 10 2017 006 655 A1 beschriebenen System der Fall ist. Dies erlaubt insbesondere den Einsatz des Systems bei Kindern. "Im Bereich zwischen" schließt hier auch einen Anschluss unmittelbar am Filter beziehungsweise am Patienteninterface ein, insbesondere im Fall einer Gesichts- oder Larynxmaske oder eines Beatmungshelmes. Überraschend hat sich gezeigt, dass der fluidische Anschluss zwischen dem Filter und dem Patienteninterface zwar die Wirkung des Totraum-Minimierungssystem mindert, die Minderung aber gering ist, weil die Totraumminimierung durch das erfindungsgemäße System nicht auf den Totraum zwischen den beiden fluidischen Anschlussstellen beschränkt ist. So hat sich gezeigt, dass ein Teil der Absaugung aus dem patientennahen Anschluss auch Gas betrifft, welches sich patientenseitig des patientennahen Anschlusses befindet. Somit kann auch der Filter erfindungsgemäß zum Einsatz kommen.

Im Falle einer nicht-invasiven Beatmung, die vorzugsweise mit einer Gesichtsmaske oder einem Beatmungshelm als Patienteninterface erfolgt, ist die zweite Pumpeinheit alternativ durch das Patienteninterface hindurch fluidisch an den Nasen- oder Rachenraum des Patienten anschließbar. Mit "hindurch" ist hier auch eine Durchführung im Bereich der Anlage des Patienteninterfaces am Körper des Patienten gemeint. Im Vergleich zu der zuvor genannten Variante (Anschluss zwischen Filter und Patienteninterface) wird somit mit dem Totraum-Minimierungssystem noch näher am Patienten abgesaugt und zurückgeführt, was die Elimination durch das Totraum-Minimierungssystem verbessert. Es wird jedoch kein Schlauch in der Luftröhre benötigt. Daneben ergibt sich ein weiterer Vorteil: Bei der nicht-invasiven Beatmung treten häufig Leckageverluste im Bereich der Dichtung des Patienteninterfaces gegenüber dem Patienten auf. Diese werden zur Aufrechterhaltung des gewünschten Druckes durch nachströmendes Beatmungsgas ausgeglichen. Durch daraus resultierende Verdünnungseffekte sind Messungen der CO2-Konzentration im Ausatemstrom meist nicht geeignet, um den wahren CO2-Gehalt des vom Patienten ausgeatmeten Gases hinreichend genau zu bestimmen. Durch den Anschluss der zweiten Pumpeinheit an den Nasen- oder Rachenraum des Patienten kann der CO2-Gehalt des von der zweiten Pumpeinheit geförderten Atemgases gemessen werden und zwar im Wesentlichen ohne die Verfälschung durch Leckageeffekte. Somit kann durch das Totraum-Minimierungssystem ein verlässlicher Wert zur Bestimmung des endexspiratorischen CO2-Partialdrucks ermittelt werden.

Beide genannte Varianten (Anschluss zwischen Filter und Patienteninterface oder Anschluss an den Nasen- oder Rachenraum des Patienten) haben die Grundidee gemeinsam, dass es für eine wirksame Eliminierung des Totraums bei der Beatmung genügen kann, wenn die zweite Pumpeinheit nicht am patientenseitigen Ende dieses Totraums, nämlich in der Lunge, sondern innerhalb der Gesamtstrecke des Totraums angeschlossen ist.

Selbst wenn das Beatmungsgerät temporär abgeschaltet und/oder abgekoppelt werden sollte, kann der beschriebene Anschluss der zweiten Pumpeinheit im Bereich des Nasen-/Rachenraumes einen wertvollen Beitrag zur Minderung von Totraumeffekten leisten.

In einer bevorzugten Ausführungsform weist das Beatmungssystem ein Absaugsystem zwischen dem Filter und dem Patienteninterface auf. Das Absaugsystem dient insbesondere dazu, einen Absaugschlauch durch das Patienteninterface in die Luftröhre zu führen um Sekrete abzusaugen. Die Erfindung sieht vorzugsweise vor, dass die zweite Pumpeinheit im Bereich zwischen dem Absaugsystem und dem Patienteninterface fluidisch angeschlossen ist. Somit liegt das Absaugsystem wie der Filter zwischen den Anschlüssen der Pumpeinheiten und wird als Totraum von dem Totraum-Minimierungssystem kompensiert.

Vorzugsweise ist die zweite Pumpeinheit derart an das Beatmungssystem fluidisch angeschlossen, dass ein Gasstrom aus der ersten Pumpeinheit in einem Winkel von 0° bis 70° und vorzugsweise zwischen 30° und 60° in das Beatmungssystem mündet. Dies wird insbesondere durch einen Konnektor sichergestellt, der die Form eines Rohrstücks mit einem schrägen Abzweig aufweist. Durch die schräge Zusammenführung der Gasströme wird ein Druckverlust durch Verwirbelungen gegenüber einer Zusammenführung im rechten Winkel verringert und somit die Wirkung des Totraum-Minimierungssystem verbessert. Insbesondere mündet der Gasstrom aus der ersten Pumpeinheit schräg in Richtung Patienteninterface, vor allem wenn exspiratorisch abgesaugt und inspiratorisch rückgeführt wird, da dann jeweils die Richtungen der Gasströme miteinander harmonieren.

Bei einer beginnend-exspiratorischen Rückführung erfolgt der fluidische Anschluss der zweiten Pumpeinheit vorzugsweise derart, dass ein Gasstrom derart aus der zweiten Pumpeinheit in das Beatmungssystem mündet, dass der Gasstrom aus der zweiten Pumpeinheit beim Einmünden einen Richtungsanteil parallel zu einem Gasstrom des Beatmungssystems hin zum Beatmungsgerät aufweist.

Für das exspiratorische Absaugen erfolgt der fluidische Anschluss der zweiten Pumpeinheit vorzugsweise derart, dass ein Gasstrom derart das Beatmungssystem zur zweite Pumpeinheit verlässt, dass der Gasstrom zur Pumpeinheit beim Verlassen einen Richtungsanteil parallel zu einem Gasstrom des Beatmungssystems hin zum Beatmungsgerät aufweist.

Bei einem starren fluidischen Anschluss widersprechen sich die beiden vorgenannten Bedingungen. Die Erfindung schlägt daher vor, dass der Anschluss über wechselnde Mündungen erfolgt, also eine erste Mündung, durch die ein Gasstrom aus der zweiten Pumpeinheit mit einen Richtungsanteil parallel zu einem Gasstrom des Beatmungssystems hin zum Beatmungsgerät in das Beatmungssystem mündet, und eine zweite Mündung, durch die ein Gasstrom derart das Beatmungssystem zur zweiten Pumpeinheit verlässt, dass der Gasstrom zur zweiten Pumpeinheit beim Verlassen einen Richtungsanteil parallel zu einem Gasstrom des Beatmungssystems hin zum Beatmungsgerät aufweist. Solche wechselnden Mündungen können insbesondere über eine Verzweigung und Rückschlagventile zwischen der Verzweigung und den Mündungen realisiert werden.

Um die Parallelität der Gasströme zu verbessern, schlägt die Erfindung vor, dass die zweite Pumpeinheit derart an das Beatmungssystem fluidisch angeschlossen ist, dass eine Mündung für einen Gasstrom von und zur zweiten Pumpeinheit durch einen in das Innere eines Konnektors ragenden Fortsatz gebildet wird. Der Konnektor ist insbesondere ein zweiter Konnektor. Der Fortsatz kann so ausgebildet sein, dass sein offenes Ende in Strömungsrichtung zum Patienten oder zum Beatmungsgerät weist. Insbesondere ist der Fortsatz ein düsenartiges, gekrümmtes Rohr. Das System kann lediglich einen solchen Fortsatz aufweisen, der insbesondere in Strömungsrichtung zum Patienten weist, oder, bei dem oben beschriebenen System mit wechselnden Mündungen, zwei Fortsätze aufweisen, deren offene Enden in entgegengesetzte Richtungen weisen.

Wie erwähnt erfolgt im Falle einer nicht-invasiven Beatmung diese vorzugsweise mit einer Gesichtsmaske oder einem Beatmungshelm als Patienteninterface. Dabei ist es bevorzugt, dass das Patienteninterface eine dichtende, insbesondere mehrfach verschließbare, Durchführungsöffnung mit Fixiermitteln für eine Fluidverbindung zwischen dem Nasen- oder Rachenraum des Patienten und der zweiten Pumpeinheit aufweist. Beispielsweise kann ein Schlauch, der an der zweiten Pumpeinheit angeschlossen ist, durch eine elastisch schließende Öffnung als Durchführungsöffnung in einer Gesichtsmaske bis zum Nasen- oder Rachenraum des Patienten reichen.

Alternativ oder zusätzlich kann vorgesehen sein, dass die Gesichtsmaske oder der Beatmungshelm als Patienteninterface einen Interfacekonnektor zum Anschluss einer Fluidverbindung zur zweiten Pumpeinheit sowie einen Patientenschlauch zum fluidischen Anschluss an den Nasen- oder Rachenraum des Patienten aufweist. Dabei kann der Patientenschlauch unmittelbar auf der Patientenseite des Patienteninterfaces an den Interfacekonnektor angeformt, oder über einen weiteren Konnektor als separates Bauteil verbunden sein. Im zweiten Fall können der Interfacekonnektor und der weitere Konnektor als ein doppelseitiger Konnektor ausgeführt sein, so dass sowohl patientenseitig als auch umgebungsseitig (im Weiteren "innen" und "außen") ein Schlauch angeschlossen werden kann. Der Anschluss kann in bekannter Weise beispielsweise durch Stecken oder Klemmen oder auch mittels Magnetkopplung erfolgen. Der Interfacekonnektor kann vor dem Anschluss der Fluidverbindung zur zweiten Pumpeinheit durch eine Schutzkappe verschlossen sein. Der Patientenschlauch kann zumindest auf einem Teil seiner Länge, insbesondere patientennah, biegesteif sein, also insbesondere durch ein zusätzliches Element oder eine zusätzliche Materialkomponente so gestaltet sein, dass er von Hand verbiegbar ist, aber unter seinem Eigengewicht seine Form behält.

Vorzugsweise weist das System einen Nasen-Prong und/oder einen atraumatischen Katheter zum fluidischen Anschluss an den Nasen- oder Rachenraum des Patienten auf. Mit einem "Nasen-Prong" ist ein nasaler Prong gemeint, wie er beispielsweise aus der High Flow Nasal Prong Sauerstofftherapie (HFNP) bekannt ist. Mit einem "atraumatischen" Katheter ist ein Katheter gemeint, der durch seine Bauart das Risiko eines Festsaugens an der Schleimhaut und/oder Verstopfens durch Sekret verringert. Beispielsweise weist der Katheter dazu wie bei einem Absaugkatheter mehrere Öffnungen und/oder einen ringförmigen Wulst um die Katheterspitze auf.

Die Erfindung wird nachfolgend anhand zweier Ausführungsbeispiele erläutert. Es zeigen:
- Figur 1: eine schematische Darstellung des ersten Ausführungsbeispiels des erfindungsgemäßen Systems in der Anwendung bei einem beatmeten Patienten;
- Figur 2: ein Ausschnitt dieses Systems in schematischer Darstellung im Bereich eines fluidischen Anschlusses des Totraum-Minimierungssystems an das Beatmungssystem;
- Figur 3: ein Ausschnitt wie in Figur 2, jedoch mit einem alternativen fluidischen Anschluss sowie mit einer Variante des alternativen Anschlusses.
- Figur 4: eine schematische Darstellung des zweiten Ausführungsbeispiels;
- Figur 5: eine schematische Detailansicht des mit V bezeichneten Ausschnitts aus Figur 4
- Figur 6: eine beispielhafte Ausführung einer Durchführungsöffnung für den zweiten Schlauch des zweiten Ausführungsbeispiels; und
- Figur 7: eine Alternative für diese Durchführungsöffnung.

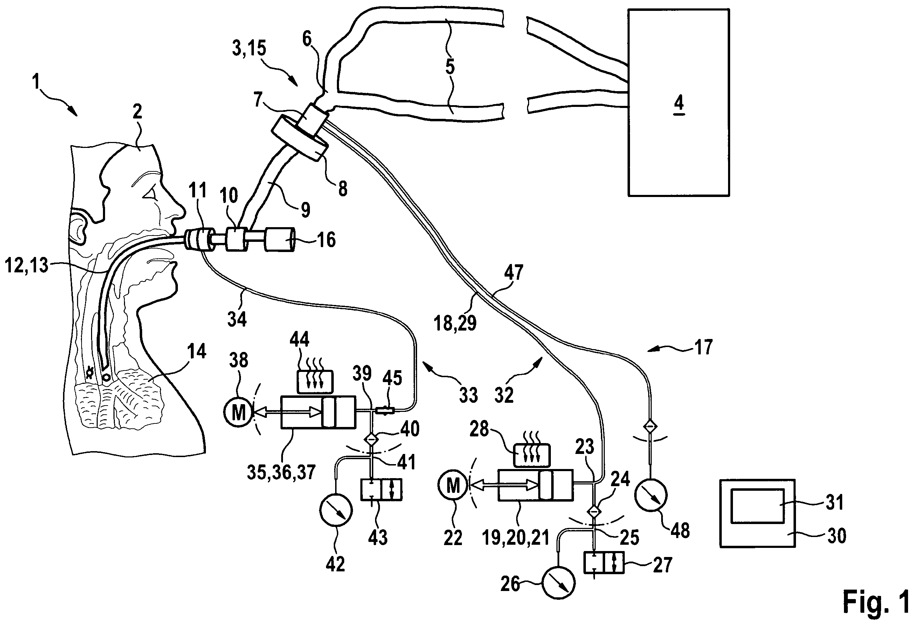

Figur 1 zeigt einen schematischen Überblick über ein erstes Ausführungsbeispiel eines erfindungsgemäßen Systems 1 in der Anwendung bei einem beatmeten Patienten 2.

Der Patient 2 wird mit einem Beatmungssystem 3 beatmet. Das Beatmungssystem 3 weist ein Beatmungsgerät 4 mit Pumpe, Steuerung, Benutzerschnittstelle und dergleichen auf, worauf es hier nicht im Detail ankommt. Von diesem Beatmungsgerät 4 gehen zwei Beatmungsschläuche 5 ab bis zu einem Y-Stück 6, an den sich nacheinander ein erster Konnektor 7, ein Filter 8 mit HME-Funktion, eine schlauchartige Verlängerung 9, ein Absaugkonnektor 10 und schließlich ein zweiter Konnektor 11 anschließt. Die Verlängerung 9 kann auch als Tubusverlängerung aufgefasst werden. An den zweiten Konnektor 11, der im Folgenden noch näher erläutert wird, ist ein Endotrachealtubus 12 als Patienteninterface 13 angeschlossen. Das Beatmungssystem 3 arbeitet in bekannter Weise, das heißt über einen Beatmungsschlauch 5, den Endotrachealtubus 12 und die dazwischen liegenden Bauteile wird Luft, Sauerstoff und/oder therapeutische Gase in die Lunge 14 des Patienten 2 gepumpt und im Wechsel dazu die verbrauchte Luft in umgekehrter Richtung, aber über den anderen Beatmungsschlauch 5 wieder abgeführt. Die Beatmungsschläuche 5 bilden zusammen mit dem Endotrachealtubus 12 und den dazwischen liegenden Bauteilen ein Leitungssystem 15 des Beatmungssystems 3. Über den Absaugkonnektor 10 ist ein Absaugsystem 16, wie es beispielweise aus der Druckschrift US 7,779,842 B1 bekannt ist, an das Leitungssystem 15 des Beatmungssystems 3 angeschlossen. Das Absaugsystem 16 ist in der Figur nur symbolisiert dargestellt, da es auf die Einzelheiten nicht ankommt.

Außerdem ist an das Beatmungssystem 3 ein Totraum-Minimierungssystem 17 angeschlossen. Das Totraum-Minimierungssystem 17 weist einen ersten Schlauch 18 auf, der an den ersten Konnektor 7 angeschlossen ist. Der erste Schlauch 18 dient der fluidischen Verbindung mit einer ersten Reservoireinheit 19 des Totraum-Minimierungssystems 17. Die erste Reservoireinheit 19 ist als eine erste Kolbenpumpe 20 ausgebildet und bildet somit gleichzeitig eine erste Pumpeinheit 21. Die erste Kolbenpumpe 20 wird von einem ersten Linearmotor 22 angetrieben, der in Figur 1 symbolisch dargestellt ist.

Der erste Schlauch 18 ist durch einen ersten Abzweig 23 unterbrochen. Der erste Abzweig 23 führt über ein erstes Filterelement 24 zu einem zweiten Abzweig 25, an dessen einem Ausgang ein erster Hochdrucksensor 26 und dessen anderem Ausgang ein erstes Be- und Entlüftungsventil 27 mit Ausgang zur Umgebung angeschlossen ist. Die Kolbenpumpe 20 ist durch ein erstes thermoelektrisches Heizelement 28 beheizbar. Außerdem ist der erste Schlauch 18 mit einer ersten thermischen Isolierung 29 umhüllt.

Die Aktoren, also der erste Linearmotor 22, das erste Heizelement 28 und das erste Be- und Entlüftungsventil 27 sind jeweils über elektrische Leitungen, die der Übersicht halber nicht dargestellt sind, mit einer Steuerung 30 verbunden. Die Steuerung 30 umfasst insbesondere eine Ein- und Ausgabeeinheit in Form einer berührungsempfindlichen Anzeige 31, ein sogenannter "Touch-Screen", zur Bedienung durch einen Anwender.

Der erste Schlauch 18 mit der ersten Pumpeinheit 21 und den an den ersten Abzweig 23 angeschlossenen Komponenten bildet einen ersten Strang 32 des Totraum-Minimierungssystems 17.

Ein zweiter Strang 33 des Totraum-Minimierungssystems 17 wird durch einen zweiten Schlauch 34, der über den zweiten Konnektor 11 fluidisch mit dem Beatmungssystem 3 verbunden ist, sowie eine zweite Reservoireinheit 35, die als zweite Kolbenpumpe 36 ausgebildet ist und somit eine zweite Pumpeinheit 37 bildet, einen zweiten Linearmotor 38, einen dritten Abzweig 39, ein zweites Filterelement 40, einen vierten Abzweig 41, einen zweiten Hochdrucksensor 42, ein zweites Be- und Entlüftungsventil 43 sowie ein zweites Heizelement 44 gebildet. Der Aufbau entspricht dem des ersten Stranges 32, jedoch ist der zweite Schlauch 34 patientenseitig vom dritten Abzweig 39 unterbrochen von einer Messküvette 45 für einen Gasanalyse-Sensor. Auch die Aktoren des zweiten Stranges 33 sowie der Sensor in der Messküvette 45 sind an die Steuerung 30 angeschlossen.

Die beiden Stränge 32, 33 ohne die beiden Linearmotoren 22, 38, die beiden Hochdrucksensoren 26, 42 und die beiden Be- und Entlüftungsventile 27, 43 bilden ein Patientenset 46, das als Einwegprodukt ausgelegt ist, was nicht ausschließt, dass einzelne Komponenten für den wiederholten Gebrauch aufbereitet, also insbesondere gereinigt und sterilisiert, werden können.

Zum Totraum-Minimierungssystem 17 gehört außerdem ein am ersten Konnektor 7 angeschlossener dritter Schlauch 47, über den ein Beatmungsdrucksensor 48 an das Beatmungssystem 3 angeschlossen ist. Auch der dritte Schlauch 47 gehört zum Patientenset 46.

Die beiden Stränge 32, 33 unterstützen die Beatmung des Patienten 2, die im Wesentlichen durch das Beatmungssystem 3 erfolgt, indem mittels der beiden Kolbenpumpen 20, 36 jeweils im Wechsel abgepumpt und zurückgeführt wird. Dabei wird am Ende der Ausatmung verbrauchtes Atemgas vom zweiten Strang 33 aus einem patientennahen Teil des Beatmungssystems 3 gesaugt, während im Wesentlichen gleichzeitig vom ersten Strang 32 frisches Gas zurückgeführt wird. Tatsächlich beginnt das Zurückführen schon etwas früher, damit im Leitungssystem 15 des Beatmungssystems 3 keine Druckminderung erfolgt, die dem Beatmungssystem 3 ein Einatmen des Patienten suggerieren würde. Durch das Absaugen wird das verbrauchte CO₂-reiche Gas aus dem patientennahen Bereich des Beatmungssystems 3 durch den zweiten Strang 33 entfernt und durch das über den ersten Strang 32 zugeführte Frischgas ersetzt.

Das vom zweiten Strang 33 abgesaugte Gas wird am Ende der folgenden Einatmung bzw. zum Beginn der folgenden Ausatmung wieder zurückgeführt, während im Wesentlichen gleichzeitig vom ersten Strang 32 frisches Gas aus dem Beatmungssystem 3 abgepumpt wird. Bezüglich der Funktionsweise des Totraum-Minimierungssystems 17 wird im Übrigen auf die Druckschrift DE 10 2017 006 655 A1 verwiesen.

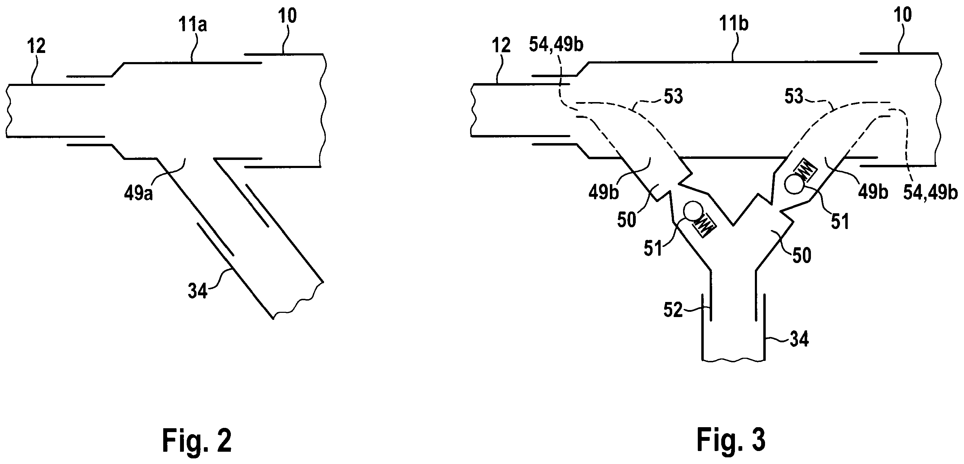

Die Figuren 2 und 3 zeigen den Anschluss des zweiten Schlauchs 34 des Totraum-Minimierungssystems 17 an das Beatmungssystem 3 in zwei alternativen Konfigurationen. Während die Figur 1 den zweiten Konnektor 11 so darstellt, dass der Gasstrom des Totraum-Minimierungssystems 17 senkrecht in den Gasstrom des Beatmungssystems 3 mündet beziehungsweise diesen senkrecht verlässt, sehen die zweiten Konnektoren 11a, 11b der Figuren 2 und 3 jeweils ein schräges Münden beziehungsweise Verlassen vor. Allgemein kann der zweite Konnektor 11, 11a, 11b als ein Rohrstück mit einem oder mehreren Abzweigen für den zweiten Schlauch 34 aufgefasst werden.

Der zweite Konnektor 11a der Figur 2 weist eine Mündung 49a schräg zum Patienten 2 hin auf, nämlich derart, dass ein Gasstrom aus der zweiten Pumpeinheit 37 in einem Winkel von etwa 50° in das Beatmungssystem 3 mündet. Dies kann auch als schräger Abzweig aufgefasst werden. Beim exspiratorischen Absaugen verlässt ein Gasstrom das Beatmungssystem 3 zur zweiten Pumpeinheit 37 derart, dass der Gasstrom zur zweiten Pumpeinheit 37 beim Verlassen einen Richtungsanteil parallel zu einem Gasstrom des Beatmungssystems 3 hin zum Beatmungsgerät 4 aufweist. Anders ausgedrückt wird der Gasstrom in dieser Situation Y-förmig aufgeteilt. Hierdurch wird das Absaugen begünstigt, weil es zu wenig Druckverlusten aufgrund von Verwirbelungen kommt. Allerdings erfolgt die Rückführung von der zweiten Pumpeinheit 37 bei einer beginnend-exspiratorischen Rückführung schräg gegen den Gasstrom des Beatmungssystem 3, was nicht optimal ist. Da die Absaugung die wichtigere Rolle einnimmt, ist dies dennoch gegenüber einer schrägen Anordnung in Richtung zum Beatmungsgerät 4 bevorzugt.

Der zweite Konnektor 11b der Figur 3 löst dies dadurch, dass er zwei Mündungen 49b aufweist, die entgegengesetzt schräg angeordnet sind, also einmal schräg zum Patienten 2 hin und einmal schräg zum Beatmungsgerät 4 hin. Zu beiden Mündungen 49b verlaufen Anschlussrohre 50, in denen jeweils ein Rückschlagventil 51 angeordnet ist und die gemeinsam Y-förmig in einen Anschlussstutzen 52 münden. Anders ausgedrückt weist der Anschlussstutzen 52 eine Verzweigung in die beiden Anschlussrohre 50 auf. An den Anschlussstutzen 52 ist der zweite Schlauch 34 angeschlossen. Die Rückschlagventile 51 sind entgegengesetzt zueinander angeordnet und zwar derart, dass beim Absaugen ein Gasstrom vom Patienten 2 kommend Y-förmig aufgeteilt wird und dabei der Gasstrom zur zweiten Pumpeinheit 37 beim Verlassen des Beatmungssystems 3 einen Richtungsanteil parallel zu einem Gasstrom des Beatmungssystems 3 hin zum Beatmungsgerät 4 aufweist. Beim Rückführen von der zweiten Pumpeinheit 37 bewirken die Rückschlagventile 51, dass der Gasstrom über die andere der beiden Mündungen 49b zum Gasstrom des Beatmungssystems 3 stößt. Der fluidische Anschluss des Totraum-Minimierungssystems 17 erfolgt also über wechselnde Mündungen 49b. Bei einer beginnend-exspiratorisch Rückführung bedeutet dies, dass der von der zweiten Pumpeinheit 37 kommende Gasstrom einen Richtungsanteil parallel zu einem Gasstrom des Beatmungssystems 3 hin zum Beatmungsgerät 4 aufweist. So wird sowohl für die Absaugung als auch für die Rückführung eine verwirbelungsarme Gasführung erreicht.

Mit punktierten Linien ist in Figur 3 außerdem eine Variante der Mündungen 49b gezeigt. Dabei reichen düsenartige, gekrümmte Fortsätze 53 von den Anschlussrohren 50 ins Innere des zweiten Konnektors 11b. Aufgrund der Krümmung der Fortsätze 53 weisen offene Enden 54 der Fortsätze 53 jeweils in entgegengesetzte Richtungen, nämlich zum einen in Richtung des Endotrachealtubus 12 und zum anderen in Richtung des Absaugkonnektors 10 und bilden so die Mündungen 49b. Hierdurch haben die zur beziehungsweise von der zweiten Pumpeinheit 37 fließenden Gasströme nicht nur einen parallelen Richtungsanteil zum Gasstrom des Beatmungssystems 3, sondern sind im Wesentlichen vollständig parallel zu diesem.

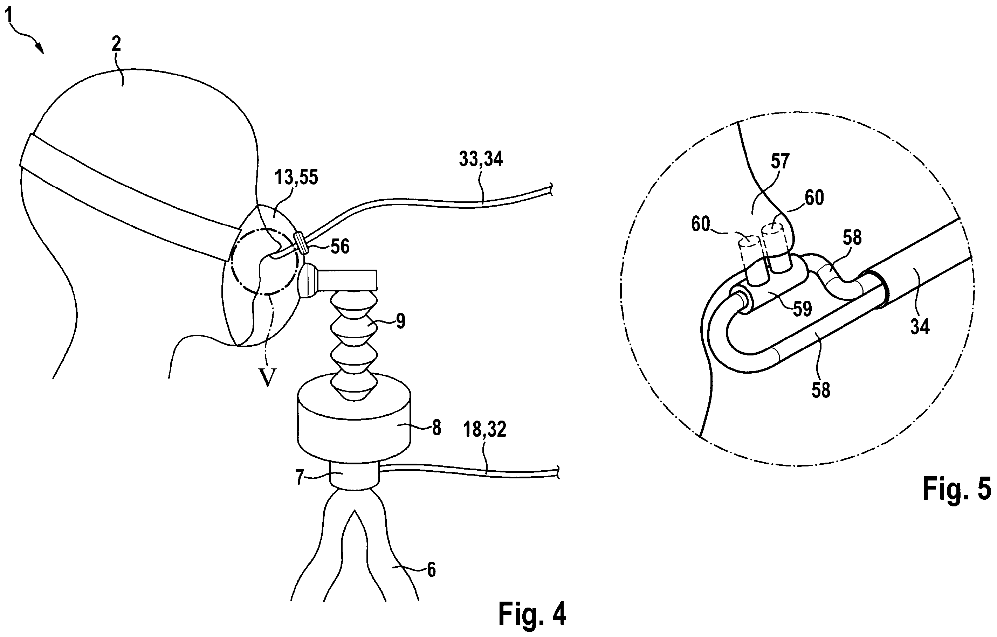

Figur 4 zeigt eine schematische Darstellung eines zweiten Ausführungsbeispiels. Viele Elemente sind gleich wie im ersten Ausführungsbeispiel, weshalb beispielsweise die Pumpeinheiten nicht dargestellt sind und ansonsten für gleiche oder sich entsprechende Elemente die gleichen Bezugsziffern verwendet sind. Ein erster Strang 32 des Totraum-Minimierungssystems ist mit einem ersten Schlauch 18 wie im ersten Ausführungsbeispiel an einem ersten Konnektor 7 des Beatmungssystems angeschlossen. Anders als im ersten Ausführungsbeispiel dient als Patienteninterface 13 jedoch eine Gesichtsmaske 55. An diese Gesichtsmaske 55 ist, wie bei der nicht-invasiven Beatmung üblich, eine Verlängerung 9 in Form eines flexiblen Schlauchs angeschlossen, an die sich ein Filter 8, der erste Konnektor 7 und ein Y-Stück 6 anschließt.

Die Gesichtsmaske 55 weist eine Durchführungsöffnung 56 für einen zweiten Schlauch 34 eines zweiten Strangs 33 des Totraum-Minimierungssystems auf. Auf die Durchführungsöffnung 56 wird im Weiteren noch eingegangen. Der zweite Schlauch 34 erstreckt sich durch die Durchführungsöffnung 56 hindurch bis in die Gesichtsmaske 55 und von dort weiter zur Nase 57 des Patienten 2. Dies ist in Figur 5 genauer dargestellt. Der zweite Schlauch 34 teilt sich innerhalb der Gesichtsmaske 55 in zwei Zuleitungen 58, die von zwei Seiten in einen Nasen-Prong 59 münden. Dieser weist zwei Auslässe 60 auf, die in die Nase 57 reichen, wobei Luft an den Auslässen 60 vorbei strömen kann. Somit ist die zweite Pumpeinheit über den zweiten Schlauch 34 durch das Patienteninterface 13 hindurch fluidisch an den Nasenraum des Patienten 2 angeschlossen beziehungsweise mit dem Nasenraum verbunden. Die Funktion entspricht dem ersten Ausführungsbeispiel.

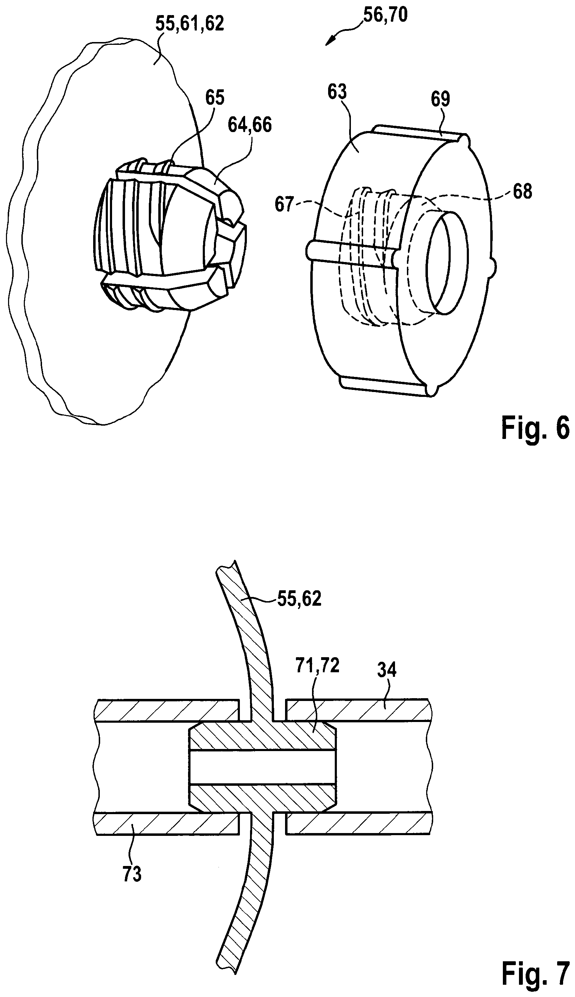

Figur 6 zeigt eine beispielhafte Ausführung der Durchführungsöffnung 56. Die Durchführungsöffnung 56 besteht aus zwei Teilen, zum einen einem Grundkörper 61, der einstückig mit einem Maskengrundkörper 62 der Gesichtsmaske 55 ist, und zum anderen einer Klemmmanschette 63. Beide Teile sind im Wesentlichen rotationssymmetrisch. Der Grundkörper 61 weist ein vierfach geschlitztes Klemmstück 64 auf, das ein Außengewinde 65 und ein konisch zulaufendes Ende 66 aufweist. Die Klemmmanschette 63 ist hülsenartig und weist ein zum Außengewinde 65 passendes Innengewinde 67 auf, an das sich eine konische Durchmesserverjüngung 68 anschließt. Über den äußeren Umfang verteilt weist die Klemmmanschette 63 vier Längswulste 69 zur besseren Handhabung auf.

Bei der Anbringung des Systems 1 am Patienten 2 wird der zweite Schlauch 34 durch den Grundkörper 61 und die Durchführungsmanschette 63 der Durchführungsöffnung 56 hindurch vom Inneren der Gesichtsmaske 55 nach außen geschoben. Sobald der Nasen-Prong 59 und die Gesichtsmaske 55 am Patienten 2 fixiert sind, kann die Position des zweiten Schlauchs 34 mittels der Durchführungsöffnung 56 fixiert werden. Dazu wird die Klemmmanschette 63 auf das Klemmstück 64 geschraubt. Das konisch zulaufende Ende 66, die Durch¬messer¬verjüngung 68 sowie die Schlitzung des Klemmstücks 64 bewirken zusammen, dass das Klemmstück 64 radial zusammengedrückt wird, wodurch der zweite Schlauch 34 geklemmt wird. Die Schlitzung ist dabei so dimensioniert, dass gleichzeitig eine Abdichtung erreicht wird. Somit bilden das Klemmstück 64 und die Klemmmanschette 63 Fixiermittel 70 für den zweiten Schlauch 34, und die Durchführungsöffnung 56 ist dichtend.

Eine Alternative zu der Durchführungsöffnung 56 zeigt Figur 7. Die Gesichtsmaske 55 weist hier einstückig einen Doppelkonnektor 71 in Form eines Rohrstücks, welches sich durch den Maskengrundkörper 62 der Gesichtsmaske 55 hindurch erstreckt beziehungsweise von diesem nach innen und außen ragt. Der äußere Teil des Doppelkonnektors 71 bildet einen Interfacekonnektor 72, auf den der zweite Schlauch 34 aufgesteckt ist. Er könnte zusätzlich mit einer Klemme als Fixiermittel gesichert sein. Auf der inneren Seite ist auf den Doppelkonnektor 71 ein Patientenschlauch 73 gesteckt. Der Patientenschlauch 73 kann wiederum mit einem Nasen-Prong oder mit einem atraumatischen Katheter verbunden sein.

### Bezugszeichenliste

- 1: System
- 2: Patient
- 3: Beatmungssystem
- 4: Beatmungsgerät
- 5: Beatmungsschlauch
- 6: Y-Stück
- 7: erster Konnektor
- 8: Filter
- 9: Verlängerung
- 10: Absaugkonnektor
- 11, 11a, 11b: zweiter Konnektor
- 12: Endotrachealtubus
- 13: Patienteninterface
- 14: Lunge des Patienten 2
- 15: Leitungssystem des Beatmungssystems 3
- 16: Absaugsystem
- 17: Totraum-Minimierungssystem
- 18: erster Schlauch
- 19: erste Reservoireinheit
- 20: erste Kolbenpumpe
- 21: erste Pumpeinheit
- 22: erster Linearmotor
- 23: erster Abzweig
- 24: erstes Filterelement
- 25: zweiter Abzweig
- 26: erster Hochdrucksensor
- 27: erstes Be- und Entlüftungsventil
- 28: erstes Heizelement
- 29: Isolierung
- 30: Steuerung
- 31: Anzeige
- 32: erster Strang des Totraum-Minimierungssystems 17
- 33: zweiter Strang des Totraum-Minimierungssystems 17
- 34: zweiter Schlauch
- 35: zweite Reservoireinheit
- 36: zweite Kolbenpumpe
- 37: zweite Pumpeinheit
- 38: zweiter Linearmotor
- 39: dritter Abzweig
- 40: zweites Filterelement
- 41: vierter Abzweig
- 42: zweiter Hochdrucksensor
- 43: zweites Be- und Entlüftungsventil
- 44: zweites Heizelement
- 45: Messküvette
- 46: Patientenset
- 47: dritter Schlauch
- 48: Beatmungsdrucksensor
- 49a, 49b: Mündung
- 50: Anschlussrohr
- 51: Rückschlagventil
- 52: Anschlussstutzen
- 53: Fortsatz
- 54: Ende des Fortsatzes 53
- 55: Gesichtsmaske
- 56: Durchführungsöffnung
- 57: Nase des Patienten 2
- 58: Zuleitung
- 59: Nasen-Prong
- 60: Auslass des Nasen-Prongs 59
- 61: Grundkörper der Durchführungsöffnung 56
- 62: Maskengrundkörper
- 63: Klemmmanschette
- 64: Klemmstück
- 65: Außengewinde
- 66: Ende des Klemmstücks 64
- 67: Innengewinde
- 68: Durchmesserverjüngung
- 69: Längswulst
- 70: Fixiermittel
- 71: Doppelkonnektor
- 72: Interfacekonnektor
- 73: Patientenschlauch

## Patentansprüche

1. System (1) zur Unterstützung des pulmonalen Gasaustauschs bei einem Patienten (2) mit
einem Beatmungssystem (3), das ein Beatmungsgerät (4), ein Y-Stück (6), einen Filter (8) und ein Patienteninterface (13) aufweist, wobei der Filter (8) fluidisch zwischen dem Beatmungsgerät (4) und dem Patienteninterface (13) angeordnet ist, und mit
einem mit dem Beatmungssystem (3) fluidisch gekoppelten Totraum-Minimierungssystem (17), das zwei separat steuerbare Pumpeinheiten (21, 37) mit je einer Reservoireinheit (19, 35) sowie eine Steuerung (30) aufweist derart,
dass eine exspiratorische, insbesondere end-exspiratorische, Absaugung von Atemgas und eine inspiratorische, insbesondere end-inspiratorische, oder beginnend-exspiratorische Rückführung des abgesaugten Atemgases durch eine zweite der beiden Pumpeinheiten (37) einstellbar ist, und dass die erste der beiden Pumpeinheiten (21) im Wesentlichen gegenläufig zur zweiten Pumpeinheit (37) arbeitet,
wobei die erste Pumpeinheit (21) im Bereich zwischen dem Beatmungsgerät (4) und dem Filter (8), insbesondere zwischen dem Y-Stück (6) und dem Filter (8), fluidisch angeschlossen ist,
**dadurch gekennzeichnet, dass** die zweite Pumpeinheit (37) im Falle einer invasiven Beatmung im Bereich zwischen dem Filter (8) und dem Patienteninterface (13) fluidisch angeschlossen ist oder, im Falle einer nicht-invasiven Beatmung, durch das Patienteninterface (13) hindurch fluidisch an den Nasen- oder Rachenraum des Patienten (2) anschließbar ist.

2. System (1) nach Anspruch 1, **dadurch gekennzeichnet, dass** das Beatmungssystem (3) ein Absaugsystem (16) zwischen dem Filter (8) und dem Patienteninterface (13) aufweist und dass die zweite Pumpeinheit (37) im Bereich zwischen dem Absaugsystem (16) und dem Patienteninterface (13) fluidisch angeschlossen ist.

3. System (1) nach Anspruch 1 oder 2, **dadurch gekennzeichnet, dass** die zweite Pumpeinheit (37) derart an das Beatmungssystem (3) fluidisch angeschlossen ist, dass ein Gasstrom aus der zweiten Pumpeinheit (37) in einem Winkel von 0° bis 70° und vorzugsweise zwischen 30° und 60° in das Beatmungssystem (3) mündet.

4. System (1) nach Anspruch 3, **dadurch gekennzeichnet, dass** ein Gasstrom derart aus der zweiten Pumpeinheit (37) in das Beatmungssystem (3) mündet, dass der Gasstrom aus der zweiten Pumpeinheit (37) beim Einmünden einen Richtungsanteil parallel zu einem Gasstrom des Beatmungssystems (3) hin zum Beatmungsgerät (4) aufweist.

5. System (1) nach Anspruch 3 oder 4, **dadurch gekennzeichnet, dass** ein Gasstrom derart das Beatmungssystem (3) zur zweiten Pumpeinheit (37) verlässt, dass der Gasstrom zur zweiten Pumpeinheit (37) beim Verlassen einen Richtungsanteil parallel zu einem Gasstrom des Beatmungssystems (3) hin zum Beatmungsgerät (4) aufweist.

6. System (1) nach Anspruch 4 und 5, **dadurch gekennzeichnet, dass** der fluidische Anschluss über wechselnde Mündungen (49b) erfolgt.

7. System (1) nach einem der vorstehenden Ansprüche, **dadurch gekennzeichnet, dass** die zweite Pumpeinheit (37) derart an das Beatmungssystem (3) fluidisch angeschlossen ist, dass eine Mündung (49b) für einen Gasstrom von und zur zweiten Pumpeinheit (37) durch einen in das Innere eines Konnektors (11b) ragenden Fortsatz (53) gebildet wird.

8. System (1) nach Anspruch 1, **dadurch gekennzeichnet, dass** das Patienteninterface (13) eine Gesichtsmaske (55) oder ein Beatmungshelm zur nicht-invasiven Beatmung ist und eine dichtende Durchführungsöffnung (56) mit Fixiermitteln (70) für eine Fluidverbindung zwischen dem Nasen- oder Rachenraum des Patienten (2) und der zweiten Pumpeinheit (37) aufweist.

9. System (1) nach Anspruch 1, **dadurch gekennzeichnet, dass** das Patienteninterface (13) eine Gesichtsmaske (55) oder ein Beatmungshelm zur nicht-invasiven Beatmung ist und
einen Interfacekonnektor (73) zum Anschluss einer Fluidverbindung zur zweiten Pumpeinheit (37) und
einen Patientenschlauch (73) zum fluidischen Anschluss an den Nasen- oder Rachenraum des Patienten (2) aufweist.

10. System (1) nach Anspruch 8 oder 9, **dadurch gekennzeichnet, dass** System (1) einen Nasen-Prong (59) und/oder einen atraumatischen Katheter zum fluidischen Anschluss an den Nasen- oder Rachenraum des Patienten (2) aufweist.

## Claims

1. A system (1) for supporting pulmonary gas exchange in a patient (2), comprising a ventilating system (3), which comprises a ventilating device (4), a Y-piece (6), a filter (8), and a patient interface (13), wherein the filter (8) is fluidically disposed between the ventilating device (4) and the patient interface (13), and comprising a dead space minimization system (17) that is fluidically coupled to the ventilating system (3) and comprises two separately controllable pump units (21, 37), each including a reservoir unit (19, 35) and a control unit (30) so that expiratory, in particular end-expiratory, aspiration of breathing gas and inspiratory, in particular end-inspiratory, or beginning-expiratory recirculation of the aspirated breathing gas by a second of the two pump units (37) can be set, and the first of the two pump units (21) essentially operates in a manner opposite the second pump unit (37), the first pump unit (21) being fluidically connected in the region between the ventilating device (4) and the filter (8), and in particular between the Y-piece (6) and the filter (8), **characterized in that** the second pump unit (37) is in the case of invasive ventilation fluidically connected in the region between the filter (8) and the patient interface (13) or, in the case of noninvasive ventilation, can be fluidically connected through the patient interface (13) to the nasal or pharyngeal space of the patient (2).

2. The system (1) according to claim 1, **characterized in that** the ventilating system (3) comprises an aspiration system (16) between the filter (8) and the patient interface (13), and the second pump unit (37) is fluidically connected in the region between the aspiration system (16) and the patient interface (13).

3. The system (1) according to claim 1 or 2, **characterized in that** the second pump unit (37) is fluidically connected to the ventilating system (3) so that a gas flow from the second pump unit (37) joins the ventilating system (3) at an angle of 0° to 70°, and preferably between 30° and 60°.

4. The system (1) according to claim 3, **characterized in that** a gas flow from the second pump unit (37) joins the ventilating system (3) so that the gas flow from the second pump unit (37), when joining, has a directional component parallel to a gas flow of the ventilating system (3) toward the ventilating device (4).

5. The system (1) according to claim 3 or 4, **characterized in that** a gas flow leaves the ventilating system (3) toward the second pump unit (37) so that the gas flow toward the second pump unit (37), when leaving, has a directional component parallel to a gas flow of the ventilating system (3) toward the ventilating device (4).

6. A system (1) according to claims 4 and 5, **characterized in that** the fluidic connection is carried out via alternating mouths (49b).

7. A system (1) according to any one of the preceding claims, **characterized in that** the second pump unit (37) is fluidically connected to the ventilating system (3) so that a mouth (49b) for a gas flow from and to the second pump unit (37) is formed by an appendage (53) protruding into the interior of a connector (11b).

8. The system (1) according to claim 1, **characterized in that** the patient interface (13) is a face mask (55) or a ventilation helmet for non-invasive ventilation and has a sealing through-passage (56) comprising fixation means (70) for a fluid connection between the nasal or pharyngeal space of the patient (2) and the second pump unit (37).

9. The system (1) according to claim 1, **characterized in that** the patient interface (13) is a face mask (55) or a ventilation helmet for non-invasive ventilation and comprises an interface connector (73) for connecting a fluid connection to the second pump unit (37) and a patient hose (73) for the fluidic connection to the nasal or pharyngeal space of the patient (2).

10. The system (1) according to claim 8 or 9, **characterized in that** the system (1) comprises a nasal prong (59) and/or an atraumatic catheter for the fluidic connection to the nasal or pharyngeal space of the patient (2).

## Revendications

1. Système (1) pour assister l'échange gazeux pulmonaire chez un patient (2), comprenant
- un système de ventilation (3), qui comprend un dispositif de ventilation (4), une pièce en Y (6), un filtre (8), et une interface patient (13), où le filtre (8) est disposé fluidiquement entre le dispositif de ventilation (4) et l'interface patient (13),
et comprenant un système de minimisation d'espace mort (17) qui est couplé fluidiquement au système de ventilation (3) et comprend deux unités de pompage (21, 37) commandables séparément, comprenant chacune une unité de réservoir (19, 35) et une unité de commande (30) de sorte qu'une aspiration expiratoire, en particulier télé-expiratoire, de gaz respiratoire et une recirculation inspiratoire, en particulier télé-inspiratoire, ou en début d'expiration du gaz respiratoire aspiré par une deuxième des deux unités de pompage (37) puisse être réglée, et la première des deux unités de pompage (21) fonctionne essentiellement de manière opposée à la deuxième unité de pompage (37), la première unité de pompage (21) étant connectée fluidiquement dans la région entre le dispositif de ventilation (4) et le filtre (8), et en particulier entre la pièce en Y (6) et le filtre (8),
**caractérisé en ce que** la deuxième unité de pompage (37) est
- dans le cas d'une ventilation invasive, connectée fluidiquement dans la région entre le filtre (8) et l'interface patient (13) ou,
- dans le cas d'une ventilation non invasive, peut être connectée fluidiquement à travers l'interface patient (13) à l'espace nasal ou pharyngé du patient (2).

2. Système (1) selon la revendication 1,
**caractérisé en ce que** le système de ventilation (3) comprend un système d'aspiration (16) entre le filtre (8) et l'interface patient (13), et la deuxième unité de pompage (37) est connectée fluidiquement dans la région entre le système d'aspiration (16) et l'interface patient (13).

3. Système (1) selon la revendication 1 ou 2,
**caractérisé en ce que** la deuxième unité de pompage (37) est connectée fluidiquement au système de ventilation (3) de sorte qu'un flux de gaz provenant de la deuxième unité de pompage (37) rejoigne le système de ventilation (3) sous un angle de 0° à 70°, et de préférence entre 30° et 60°.

4. Système (1) selon la revendication 3,
**caractérisé en ce qu'**un flux de gaz provenant de la deuxième unité de pompage (37) rejoigne le système de ventilation (3) de sorte que le flux de gaz provenant de la deuxième unité de pompage (37), lors de la jonction, ait une composante directionnelle parallèle à un flux de gaz du système de ventilation (3) vers le dispositif de ventilation (4).

5. Système (1) selon la revendication 3 ou 4,
**caractérisé en ce qu'**un flux de gaz quitte le système de ventilation (3) vers la deuxième unité de pompage (37) de sorte que le flux de gaz vers la deuxième unité de pompage (37), lors de la sortie, ait une composante directionnelle parallèle à un flux de gaz du système de ventilation (3) vers le dispositif de ventilation (4).

6. Système (1) selon les revendications 4 et 5,
**caractérisé en ce que** la connexion fluidique est réalisée via des embouchures alternées (49b).

7. Système (1) selon l'une quelconque des revendications précédentes,
**caractérisé en ce que** la deuxième unité de pompage (37) est connectée fluidiquement au système de ventilation (3) de sorte qu'une embouchure (49b) pour un flux de gaz provenant de et vers la deuxième unité de pompage (37) soit formée par un appendice (53) faisant saillie dans l'intérieur d'un connecteur (11b).

8. Système (1) selon la revendication 1,
**caractérisé en ce que** l'interface patient (13) est un masque facial (55) ou un casque de ventilation pour ventilation non invasive et présente un passage traversant étanche (56) comprenant des moyens de fixation (70) pour une connexion fluidique entre l'espace nasal ou pharyngé du patient (2) et la deuxième unité de pompage (37).

9. Système (1) selon la revendication 1,
**caractérisé en ce que** l'interface patient (13) est un masque facial (55) ou un casque de ventilation pour ventilation non invasive et comprend un connecteur d'interface (73) pour connecter une connexion fluidique à la deuxième unité de pompage (37) et un tuyau patient (73) pour la connexion fluidique à l'espace nasal ou pharyngé du patient (2).

10. Système (1) selon la revendication 8 ou 9,
**caractérisé en ce que** le système (1) comprend une canule nasale (59) et/ou un cathéter atraumatique pour la connexion fluidique à l'espace nasal ou pharyngé du patient (2).
